# EUROPEAN PATENT APPLICATION

(11) **EP 2 599 450 A1**
(43) Date of publication of application: **05.06.2013**
(21) Application number: 11191398.4
(22) Date of filing: 30.11.2011
(51) Int. Cl.: A61B 17/064, A61B 17/122, A61B 17/128

(54) **Obliteration device**

(71) Applicant: Aldaf Holding B.V., 1277 BR Huizen (NL)
(72) Inventor: Visscher, John Anthony, 3755 LC Eemnes (NL); Kalmann, Menno, 3755 LC Eemnes (NL)
(74) Representative: Jansen, Cornelis Marinus

(57) **Abstract**

The invention relates to an obliteration device (10) for enabling a substantially full obliteration of a vascular conduit, the device comprising a clip (2, 3) and an applier (1, 6) for transcutaneously introducing the clip, the clip being provided with a first working element (2) extending in use in a substantially flat first plane and a second working element (3) extending in use in a substantially flat second plane, the first plane and the second plane being substantially parallel to each other, wherein the first working element and the second working element are manufactured from a shape memory metal enabling to store the clip in the applier in a substantially elongated state (3).

## Description

### FIELD OF THE INVENTION

The invention relates to a transluminal obliteration device for enabling a substantially full obliteration of a vascular conduit.

The invention further relates to a method of transluminal obliteration of a vascular conduit.

### BACKGROUND OF THE INVENTION

An embodiment of an obliteration device suitable for obliterating a vascular conduit is known from WO 2011/058334.

The known device comprises an applicator with an internal chamber adapted for storing a resiliently deformed wire of a shape memory alloy. In use, the applicator is provided near a vascular conduit conceived to be obliterated and the wire is released. Due to the forces urging the deformed wire to return in its shape, the wire starts to curl and by doing so is wounded about the conduit.

It is a disadvantage of the known device that the process of returning the wire in its original state takes place uncontrollably. Therefore, wire misplacement with regard to a target region of the vascular conduit may occur. More in particular, the wire may curl back accidentally without even engaging the vascular conduit.

### SUMMARY OF THE INVENTION

It is an object of the invention to provide a transluminal obliteration device which enables an accurate and a reliable obliteration of a vascular conduit. More in particular, it is an object of the invention to provide a transluminal obliteration device wherein such accurate and reliable obliteration may be effectuated transcutaneously.

To this end, the transluminal obliteration device for enabling a substantially full obliteration of a vascular conduit according to the invention, comprises a clip and an applier for transcutaneously introducing the clip, the clip being provided with a first working element extending in use in a substantially flat first plane and a second working element extending in use in a substantially flat second plane, the first plane and the second plane being substantially parallel to each other, wherein the first working element and the second working element are manufactured from a shape memory metal enabling to store the clip in the applier in a substantially elongated state.

It will be appreciated that the term 'clip' may not be interpreted in a limitative way regarding any physical property, such as length, configuration, two- or three-dimensional nature and so on. Specific features of the clip are defined in the dependent claims. More in particular, the term clip may be interchanged with the term 'closing device', 'vessel stapler', 'occluding body' and so on.

The technical measure of the invention is based on the insight that a substantial improvement of a current methodology of treating varicose veins may be achieved when a deteriorated vascular conduit may be substantially fully occluded for redirecting the venous blood flow. The occlusion may be carried out transluminally using a transcutaneous port, which substantially simplifies the procedure and reduced the risks for a patient, compared to the standard procedure of removing the deteriorated veins.

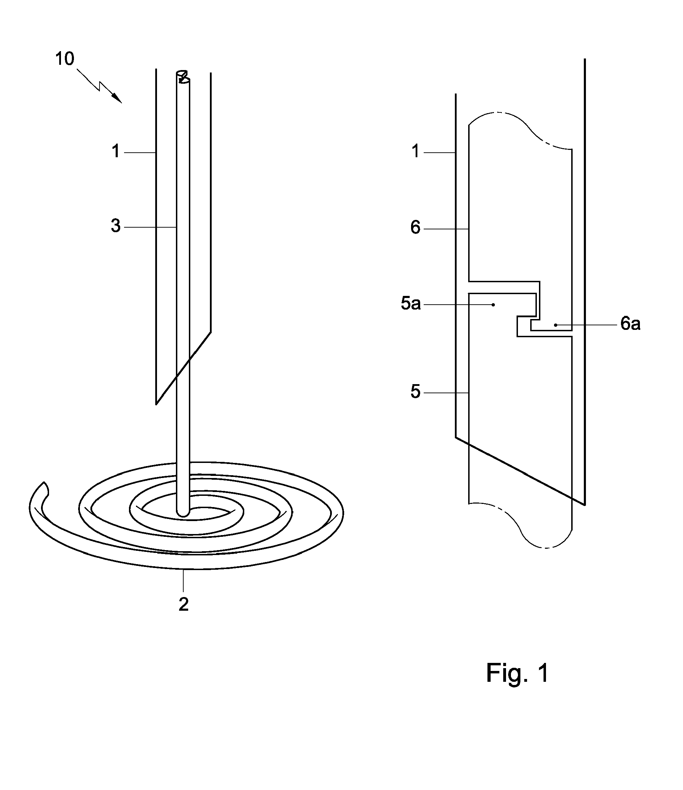

More in particular, it is found that by implementing the occluder as a wire-like element from a shape memory metal, it may be stored in a substantially elongated condition inside a delivery unit. An interstitial needle is found to be a particularly suitable body for storing and delivering the occluding body according to the invention. The transluminal occluding device according to the invention will be discussed in more detail with reference to Figure 1.

A tissue fastener using a body manufactured from a shape memory alloy is known from WO 02/19923. The known fastener is provided inside a needle and is used to fit two pieces of tissue during a surgical intervention.

When the known fastener is released from the needle it forms a spiral body extending in a direction substantially transverse to a plane of the affixed tissues, wherein the respective loops of the resulting spiral are stacked on top of each other.

The known fastener has a disadvantage that undesirable tissue damaging may occur when the respective loops of the spiral are not perfectly aligned on top of each other. Secondly, should the resulting spiral body be obliquely affixed with respect to the tissue, an imperfect connection between the respective tissue layers may occur. Finally, the known fastener is not suitable to be applied to a vascular conduit having a substantial blood pressure.

In an embodiment of the device according to the invention, the first working element and/or the second working element are manufactured from wire of the shape memory metal.

It is found to be particularly suitable to use a body manufactured from a shape memory metal for implementing a suitable occluder. More in particular, it is found that a preferred occluder is substantially flat and occludes the vein by a contracting (pulling) force which is built-in the occluder by allowing it to have a first working element and a second working element which are in use attracted to each other.

In a further embodiment of the device according to the invention, the first working element and/or the second working element are circular, linear or spiral shaped.

It is found that a particularly good obliteration of a vessel may be achieved when the working elements are implemented as a flat circular, linear or spiral bodies.

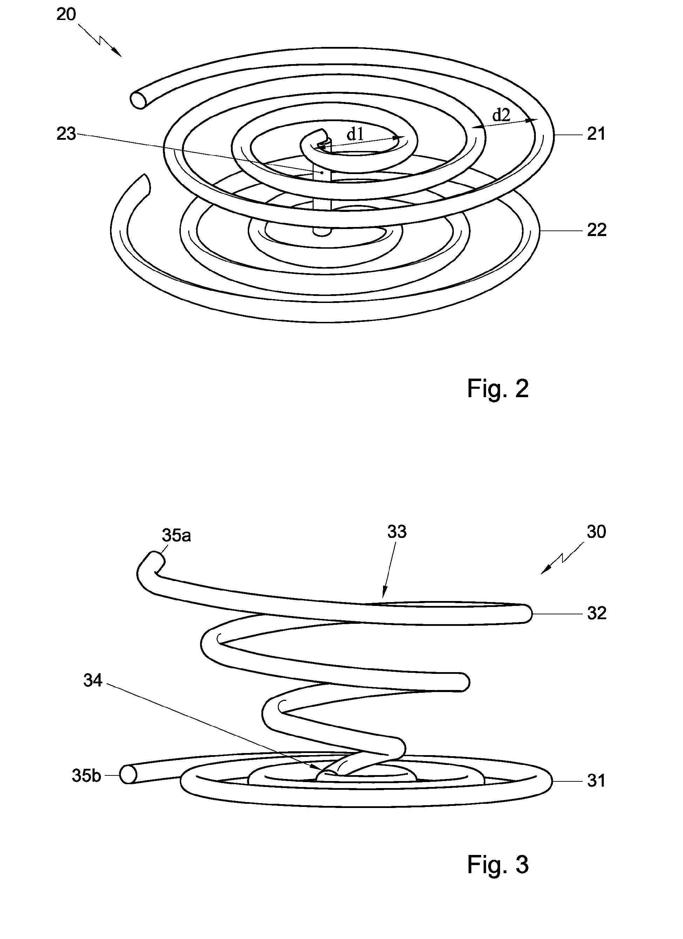

More details on these embodiments will be presented with reference to Figure 2.

In a still further embodiment of the device according to the invention the first working element and/or the second working element are spiral shaped, wherein the spiral is flat and has windings of increasing diameter.

It is found that a particular improvement of the quality of the resulting occlusion may be achieved when the first working element and the second working element are implemented as respective spirals having increasing loop diameters. In a preferred embodiment, the spirals are interconnected substantially in a center of the resulting circles and the loops adjacent the connecting element have the minimal diameter.

It is found that such embodiment has a specific advantage. When the lower spiral is deployed and is supporting the distal wall of the treated vein, the upper spiral, being delivered from the applier, projects with a substantial force as a cone towards the deployed working element. This cooperation of the deployed and therefore static lower working element with the deploying projectile of the upper working element forces the distal and the proximal walls of the lumen towards each other substantially increasing occluding reliability and accuracy.

In addition, it is noted that because the surface area of the working elements is substantially larger that the height of the clip in use, the clip will auto-direct itself towards a substantially parallel orientation with respect to the walls of the vein. This effect still further improves that accuracy and the reliability of the occluding device according to the invention.

In a still further embodiment of the device according to the invention the direction of the spiral winding of the first working element is opposite to the direction of the spiral winding of the second working element.

It is found that the working elements may be advantageously supplementary to each other when the respective spirals are wound in the opposite directions.

It is found that good occluding effects may be reached when a distance between the consecutive spiral windings of a working element is substantially constant. However, it is also possible that the distance between the consecutive spiral windings of one working element is increasing towards the periphery of the respective working element. The latter embodiment may be advantageous when a substantial force has to be induced for urging the distal wall to the proximal wall of the lumen.

In a still further embodiment of the device according to the invention, the applier comprises an interstitial needle for housing the clip in the substantially elongated state and a plunger adapted to deliver the clip from the needle at an ejection site.

It is found to be substantially advantageous to provide a plunger inside the applier for delivering the elongated occluder. Preferably, the plunger is adapted to deliver the clip in at least two delivery steps. In an advantageous embodiment, a first delivery step relates to a delivery of a first working element and second delivery step relates to a delivery of the second working element.

It is found to be advantageous to first suitably deploy the first working element (i.e. the working element conceived to cooperate with a distal wall of the lumen). When the first working element is deployed, the clip is preferably still affixed to the plunger. Accordingly, the first working element may be used as an anchor for pulling the distal wall of the lumen towards the proximal wall of the lumen prior to deploying the second working element. When the second working element is deployed the vessel is occluded sufficiently and reliably.

In a preferred embodiment the plunger is mechanically engaged with the clip for enabling handling of the vascular conduit with a deployed second working element. However, other ways of interconnecting the plunger to the clip are envisaged as well.

In a still further embodiment of the device according to the invention, the terminal portions of the first working element and the second working element are non-traumatic.

It is found that it is possible to reduce as risk of tissue damage of the lumen wall or the surrounding tissue by suitably adapting the terminal ends of the working elements. For example, it is possible that these ends are made blunt, or are shaped to face inwardly with respect to the shape of the working element. More in particular, the ends may be coated with a suitable material to make them soft.

The method of transluminal obliteration of a vascular conduit according to an aspect of the invention relates to a procedure using:
- an obliteration device for enabling a substantially full obliteration of a vascular conduit, the device comprising a clip and an applier for transluminal introduction of the clip, the clip being provided with a first working element extending in use in a substantially flat first plane and a second working element extending in use in a substantially flat second plane, the first plane and the second plane being substantially parallel to each other, wherein the first working element and the second working element are manufactured from a shape memory metal enabling to store the clip in the container in a substantially elongated state in an applier and
- an applier comprising a needle adapted for delivering the clip at an ejection site.

The method according to an aspect of the invention comprises the steps of:
- introducing the needle near the vascular conduit selected for obliteration;
- introducing the needle through the vascular conduit beyond a distal wall of the lumen;
- releasing the first working element to engage the distal wall;
- releasing the second working element to engage the proximal wall thereby obliterating the vascular conduit.

In an embodiment of the method according to an aspect of the invention the following further steps are envisaged:
- pulling the applier with the deployed first working element for positioning the distal wall of the conduit adjacent the proximal wall of the conduit,
- releasing the second working element for obliterating the vascular conduit.

These and other aspects of the invention will be discussed with reference to drawings wherein like reference signs correspond to like elements. It will be appreciated that the drawings are presented for illustrative purposes only and may not be used for limiting the scope of the appended claims.

### BRIEF DESCRIPTION OF THE DRAWINGS

Figure 1 presents in a schematic way an embodiment of an obliteration device according to an aspect of the invention.
Figure 2 presents in a schematic way a further embodiment of the first working element and the second working element.
Figure 3 presents in a schematic way an embodiment of interaction between the working elements upon deployment.
Figure 4 presents in a schematic way a further embodiment of the applier of the obliteration device according to an aspect of the invention.

### DETAILED DESCRIPTION OF THE DRAWINGS

Figure 1 presents in a schematic way an embodiment of a transluminal obliteration device according to an aspect of the invention. The said obliteration device 10 is adapted to be used to deploy an obliterating clip 2, 3 transcutaneously.

For this purpose the transluminal obliterating device comprises a needle 1 wherein the clip is arranged substantially in an elongated configuration. The clip 2, 3 is manufactured from a shape memory metal, such as Nitinol. Accordingly, the clip may be deformed to assume a substantially stretched longitudinal configuration, such as a distal portion 3 shown in Figure 1. When the clip emerges from the confinement, it resumes its defined configuration 2. It will be appreciated that in this figure for clarity reasons a first working element of the clip is shown deployed (see item 2) whereas the second working element is shown in its stretched, elongated configuration (3). Upon storage in the needle both working elements are substantially stretched and upon deployments both working elements are substantially flat and extend in respective parallel planes.

It is found that the clip according to the invention is advantageous as it provides a secure obliteration of a vessel by allowing two substantially flat working elements to exert a contracting force on the walls of the vessel.

It will be appreciated that the working elements may have different configurations. For example, a working element may be configured as a circle, ellipse, a cross, a line, a flat spiral, or even a flat area. These shapes are all possible due to the fact that the shape memory metals are highly deformable and/or foldable.

A particular improvement of the occlusion procedure may be achieved when allowing for a so-called two-stepped deployment process. For example, when a distal portion 5a of the distal working element 5 us engaged with a plunger 6 provided in the needle one, not only a controlled deployment of the working elements may be achieved, but also a manipulation of the vessel's wall using a deployed first working element may be achieved.

In a preferred embodiment the distal portion 5a of the second working element is mechanically engaged with a portion of the plunger 6a. Accordingly, the plunger may be pushed to allow for deployment of only the first working element. It will be appreciated that both the manipulation of the needle 1 as well as the deployment of the working elements may be carried out under guidance of the ultrasound for real-time visualization of the needle, the vessel's walls and the emerging and deploying working elements.

Accordingly, it may be controllably achieved that only the first working element is deployed. Because the distal end of the clip is engaged to the plunger, the plunger may be pulled back thereby closing the vessel by allowing the distal wall of the lumen to approach the proximal wall of the lumen.

For example, when under an ultrasound guidance, it is seen that the vessel is duly closed, the second working element may be released thereby firmly obliterating the vessel. More details on the dynamic interaction between the working elements are given with reference to Figure 3.

Figure 2 presents in a schematic way a further embodiment of the first working element and the second working element. It this particular embodiment spiral substantially flat working elements are discussed. It will be appreciated, however, that any other suitable flat body may be used, including but not limited to a circular body, a cross, a line, an area, a frame.

In this particular embodiment the clip 20 comprises a first working element 22 conceived to be released first from the needle of the applier. The first working elements 22 may be connected to the second working elements 21 1 by a connection element 23. It will be appreciated, however, that the length of the connection element 23 may be sub-millimeter. Alternatively, the first working element may undergo a smooth transition into the second working element.

Preferably, the respective spirals of the first working element and the second working element are wound in opposite directions having respective origins substantially in a center of the thus formed shape. Further, it is possible that the distance d1, d2 between the subsequent loops of the spirals is substantially the same. However, it is also possible that the inner windings have a smaller inter-wire spacing d1 than the spacing d2 of the peripheral wires. It is found that by allowing of a denser looping about the center of the spiral an increased pulling force may be built-in between the first working element and the second working element.

It will be appreciated that at least because the individual spirals extend in a substantially horizontal plane the total area of a clamping body is substantially increased with respect to an embodiment shown in W02011/058334, which enables effectuating a substantially improved obliterating action.

Figure 3 presents in a schematic way an embodiment of interaction between the working elements upon deployment 30. In this figure dynamics of the deploying second working element 32 is schematically shown. Thus, the first working element 31 is pre-deployed and is abutting the distal wall of the lumen (not shown for clarity). The first working element extends along a substantially flat plane.

In the area 34 the first working element transits into the second working element 32 which has been just released from the needle (not shown). When the second working element resumes its original pre-defined shape, it approaches the first working element as a cone-shaped projectile affixing the proximal wall of the lumen towards the distal wall of the lumen. As a result, the first working element dwells at an outer surface of the distal wall of the lumen and the second working element dwells at the outer surface of the proximal wall of the lumen.

It will be appreciated that it is possible that the first working element and the second working element are fully dwelling at the outer extremity of the lumen. However, it is also possible that a portion of any spiral is extending inside the lumen. This arrangement may be expected in thicker lumens or in lumens which are substantially deformed. In general, the outer diameter of any working element may be as large as the diameter of the lumen. It is found that these geometrical considerations enable secure and reliable transluminal vessel occlusion.

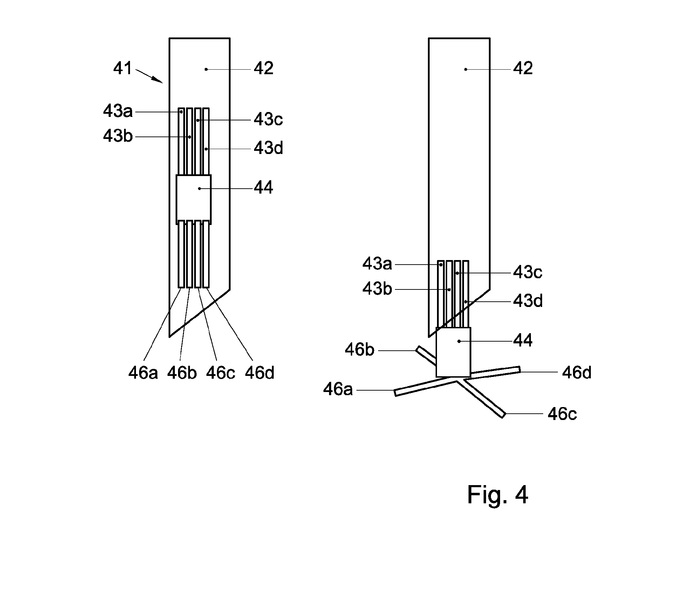

Figure 4 presents in a schematic way a further embodiment of the applier of the obliteration device according to an aspect of the invention. In this particular embodiment a cross-shaped clip is shown. Accordingly, the cross-shaped clip may be stored in a needle 42. The cross-shape may be embodied by a number of shape memory elements (for example, wires) 43a, 43b, 43c, 43d which may be interconnected by a holder 44. The first working element is formed by the other ends 46a, 46b, 46c, 46d of the shape memory elements. It will be appreciated that the ends 43a, 43b, 43c, 43d may be formed from the same wire as the ends 46a, 46b, 46c, 46d. However, it is also possible that the ends 43a, 43b, 43c, 43d and 46a, 46b, 46c, 46d do not form respective mutual elements.

Upon deployment, the elements 46a, 46b, 46c, 46d resume their original shape, which may resemble a cross, or any other desirable closed or open structure. The ends 43a, 43b, 43c, 43d may resume the same or a different shape upon deployment. It will be appreciated that the ends 43a, 43b, 43c, 43d and 46a, 46b, 46c, 46d may be rotated with respect to each other along a vertical axis, or, alternatively, the ends 43a, 43b, 43c, 43d and 46a, 46b, 46c, 46d may be configured to match and overlap in space.

While specific embodiments have been described above, it will be appreciated that the invention may be practiced otherwise than as described. Moreover, specific items discussed with reference to any of the isolated drawings may freely be inter-changed supplementing each outer in any particular way. The descriptions above are intended to be illustrative, not limiting. Thus, it will be apparent to one skilled in the art that modifications may be made to the invention as described in the foregoing without departing from the scope of the claims set out below.

## Claims

1. A transluminal obliteration device for enabling a substantially full obliteration of a vascular conduit, the device comprising a clip and an applier for transcutaneously introducing the clip, the clip being provided with a first working element extending in use in a substantially flat first plane and a second working element extending in use in a substantially flat second plane, the first plane and the second plane being substantially parallel to each other, wherein the first working element and the second working element are manufactured from a shape memory metal enabling to store the clip in the applier in a substantially elongated state.

2. The device according to claim 1, wherein the first working element and/or the second working element are manufactured from wire of the shape memory metal.

3. The device according to claim 1 or 2, wherein the first working element and/or the second working element are circular, linear or spiral shaped.

4. The device according to claim 3, wherein the first working element and/or the second working element are spiral shaped, wherein the spiral is flat and has windings of increasing diameter.

5. The device according to claim 4, wherein the direction of the spiral winding of the first working element is opposite to the direction of the spiral winding of the second working element.

6. The device according to claim 4 or 5, wherein the distance between the consecutive spiral windings is substantially constant.

7. The device according to claim 4 or 5, wherein the distance between the consecutive spiral windings is increasing towards the periphery of the respective working element.

8. The device according to any one of the preceding claims, wherein the applier comprises an interstitial needle for housing the clip in the substantially elongated state and a plunger adapted to deliver the clip from the needle at an ejection site.

9. The device according to claim 8, wherein the plunger is adapted to deliver the clip in at least two delivery steps.

10. The device according to claim 9, wherein a first delivery step relates to a delivery of a first working element and second delivery step relates to a delivery of the second working element.

11. The device according to claim 8, 9 or 10, wherein the plunger is mechanically engaged with the clip for enabling handling of the vascular conduit with a deployed second working element.

12. The device according to any one of the preceding claims, wherein the terminal portions of the first working element and the second working element are non-traumatic.

13. The device according to claim 12, wherein the terminal portions of the first working element and the second working element are bended towards a respective centre of a spiral.

14. A method of transluminal obliteration of a vascular conduit using:
- an obliteration device for enabling a substantially full obliteration of a vascular conduit, the device comprising a clip and an applier for transluminal introduction of the clip, the clip being provided with a first working element extending in use in a substantially flat first plane and a second working element extending in use in a substantially flat second plane, the first plane and the second plane being substantially parallel to each other, wherein the first working element and the second working element are manufactured from a shape memory metal enabling to store the clip in the container in a substantially elongated state in an applier and
- an applier comprising a needle adapted for delivering the clip at an ejection site,
the method comprising the steps of:
- introducing the needle near the vascular conduit selected for obliteration;
- introducing the needle through the vascular conduit beyond a distal wall of the lumen;
- releasing the first working element to engage the distal wall;
- releasing the second working element to engage the proximal wall thereby obliterating the vascular conduit.

15. The method according to claim 14, wherein the method further comprises the steps of:
- pulling the applier with the deployed first working element for positioning the distal wall of the conduit adjacent the proximal wall of the conduit,
- releasing the second working element for obliterating the vascular conduit.
